# FASCICULE DE BREVET EUROPEEN

(11) **EP 2 183 230 B1**
(45) Date de publication et mention de la délivrance du brevet: **13.11.2013**
(21) Numéro de dépôt: 08786531.7
(22) Date de dépôt: 28.07.2008
(51) Int. Cl.: C07D 271/12, A61K 31/4245, C09K 11/06, G01N 33/533, G01N 33/574

(54) **NOUVEAUX DÉRIVÉS FLUORESCENTS DE POLYAMINES, LEUR PROCÉDÉ DE PRÉPARATION ET LEURS APPLICATIONS EN TANT QU'OUTILS DE DIAGNOSTIC DANS LE TRAITEMENT DES TUMEURS CANCÉREUSES.**
NEUE FLUORESZENTE DERIVATE VON POLYAMIN, VERFAHREN ZU DEREN HERSTELLUNG UND DEREN ANWENDUNG ALS DIAGNOSEWERKZEUGE BEI DER BEHANDLUNG VON KREBSARTIGEN TUMOREN
NOVEL FLUORESCENT DERIVATIVES OF POLYAMINE, METHOD FOR PREPARING SAME AND APPLICATIONS THEREOF AS DIAGNOSIS TOOLS IN THE TREATMENT OF CANCEROUS TUMOURS

(30) Priorité: 26.07.2007 FR 0756743
(43) Date de publication de la demande: 12.05.2010
(73) Titulaire: Pierre Fabre Médicament, 92100 Boulogne-Billancourt (FR)
(72) Inventeur: ANNEREAU, Jean-Philippe, F-31400 Toulouse (FR); BARRET, Jean-Marc, F-81100 Castres (FR); GUMINSKI, Yves, F-81090 Lagarrigue (FR); IMBERT, Thierry, F-81290 Viviers Les Montagnes (FR)
(74) Mandataire: Regimbeau
(86) Numéro de dépôt international: PCT/EP2008/059890
(87) Numéro de publication internationale: WO 2009/013360

(56) Documents cités:
- WO-A-2004/093874
- WO-A-2005/100363
- US-B1- 6 172 261
- TOYO'OKA, TOSHIMASA ET AL: "Precolumn fluorescence tagging reagent for carboxylic acids in high-performance liquid chromatography: 4-substituted-7-aminoalkylamino- 2,1,3-benzoxadiazoles" JOURNAL OF CHROMATOGRAPHY , 588(1-2), 61-71 CODEN: JOCRAM; ISSN: 0021-9673, 1991, XP002470764
- PRADOS, P. ET AL: "4-N,N-Dimethylaminosulfonyl-7-N-(2-aminoe thyl)aminobenzofurazan as a new precolumn fluorescence derivatization reagent for carboxylic acids (fatty acids and drugs containing a carboxyl moiety) in liquid chromatography" ANALYTICA CHIMICA ACTA , 344(3), 227-232 CODEN: ACACAM; ISSN: 0003-2670, 1997, XP002470765
- ONODA, MAKI ET AL: "A Photoinduced Electron-Transfer Reagent for Peroxyacetic Acid, 4-Ethylthioacetylamino-7- phenylsulfonyl-2,1,3-benzoxadiazole, Based on the Method for Predicting the Fluorescence Quantum Yields" ANALYTICAL CHEMISTRY , 74(16), 4089-4096 CODEN: ANCHAM; ISSN: 0003-2700, 2002, XP002470766
- TALIANI, SABRINA ET AL: "New Fluorescent 2-Phenylindoleglyoxylamide Derivatives as Probes Targeting the Peripheral-Type Benzodiazepine Receptor: Design, Synthesis, and Biological Evaluation" JOURNAL OF MEDICINAL CHEMISTRY , 50(2), 404-407 CODEN: JMCMAR; ISSN: 0022-2623, 2007, XP002470767
- DATABASE CA [Online] CHEMICAL ABSTRACTS SERVICE, COLUMBUS, OHIO, US; TOYOOKA, TOSHITADA ET AL: "Novel fluorescent chelating agent, and method for measuring metal ion" XP002470772 extrait de STN Database accession no. 2004:154613 & JP 2004 061476 A (TOKYO KASEI KOGYO K. K., JAPAN) 26 février 2004 (2004-02-26)
- DATABASE CA [Online] CHEMICAL ABSTRACTS SERVICE, COLUMBUS, OHIO, US; ONODA, MAKI ET AL: "The effects of spacer length on the fluorescence quantum yields of the benzofurazan compounds bearing a donor-acceptor system" XP002470773 extrait de STN Database accession no. 2002:195293 -& LUMINESCENCE , 17(1), 11-14 CODEN: LUMIFC; ISSN: 1522-7235, 2002, XP002470768
- OHBA, YUSUKE ET AL: "Synthesis of N-functionalized oleamide derivatives" TETRAHEDRON , 63(18), 3754-3761 CODEN: TETRAB; ISSN: 0040-4020, 2007, XP022009159 cité dans la demande
- DATABASE CA [Online] CHEMICAL ABSTRACTS SERVICE, COLUMBUS, OHIO, US; TOYOOKA, TOSHIMASA ET AL: "4-(Aminosulfonyl)-2,1,3-benzoxadiazole derivatives as precolumn fluorogenic tagging reagents for carboxylic acids in high-performance liquid chromatography" XP002470774 extrait de STN Database accession no. 1991:484549 & ANALYST (CAMBRIDGE, UNITED KINGDOM) , 116(6), 609-13 CODEN: ANALAO; ISSN: 0003-2654, 1991,
- P.B.GHOSH AND CO: "Potential antileukimec and immunosuppressive drugs. Pepartion an in vitro pharmacological activity of some benzo-2,1,3-oxadiazoles and their N-oxides" JOURNAL OF THE MEDICINAL CHEMISTRY, vol. 11, no. 2, 1968, pages 305-311, XP002470769
- H.HEBERER AND CO: "Derivatisierung von aminen mit 4-substituierten 7-nitrobenz-2,1,3-oxadiazolen" JOURNAL FÜR PRAKTISCHE CHEMIE, vol. 327, no. 3, 1985, pages 487-504, XP002470770
- DUCKER, CHARLES E. ET AL: "In vitro and cellular assays for palmitoyl acyltransferases using fluorescent lipidated peptides" METHODS (SAN DIEGO, CA, UNITED STATES) , 40(2), 166-170 CODEN: MTHDE9; ISSN: 1046-2023, 2006, XP005669649
- VILLA, ANNA M. ET AL: "Choline and phosphatidylcholine fluorescent derivatives localization in carcinoma cells studied by laser scanning confocal fluorescence microscopy" EUROPEAN JOURNAL OF CANCER , 41(10), 1453-1459 CODEN: EJCAEL; ISSN: 0959-8049, 2005, XP004936238
- ROBSON, CLAIRE ET AL: "Chemical synthesis and biological properties of novel fluorescent antifolates in Pgp- and MRP-overexpressing tumor cell lines" BIOCHEMICAL PHARMACOLOGY , 56(7), 807-816 CODEN: BCPCA6; ISSN: 0006-2952, 1998, XP002224958
- WARE, BENNIE R. ET AL: "Interaction of a fluorescent spermine derivative with a nucleic acid polyion" LANGMUIR , 4(2), 458-63 CODEN: LANGD5; ISSN: 0743-7463, 1988, XP002470771 cité dans la demande

## Description

Le marquage des biomolécules et le suivi des phénomènes biologiques demandent l'élaboration de sondes fluorescentes qui sont devenues des outils incontournables pour les études structurelles et fonctionnelles des milieux biologiques.

Une sonde fluorescente est une molécule conjuguée, composée d'un groupement fluorophore ou fluorochrome attaché de façon covalente à un groupement ayant des propriétés d'interactions spécifiques avec l'environnement biologique adéquat.

Cette sonde fluorescente possède la propriété d'absorber l'énergie lumineuse qui lui est adressée à une longueur d'onde bien définie, constituant ainsi la phase d'excitation, puis de restituer cette énergie sous forme de lumière fluorescente, appelée phase d'émission. Ainsi, après excitation, une fois l'énergie du photon absorbée, la molécule se trouve dans un état électroniquement excité, et retourne alors à l'état d'énergie fondamentale en émettant un photon. Cette émission de photon, phénomène de fluorescence, se fait à une longueur d'onde légèrement supérieure à la longueur d'onde d'excitation. On peut alors enregistrer un spectre de fluorescence qui est spécifique de la molécule.

Il est alors possible de localiser une sonde fluorescente dans un milieu biologique à l'aide de microscopes à fluorescence ou d'un cytomètre en flux en excitant la préparation à une longueur d'onde appropriée.

Il existe un grand choix de fluorochromes, comme par exemple les dérivés de fluoresceine ou de rhodamine. Tous ne sont pas adaptés au suivi biologique. Les caractéristiques essentielles sont : les valeurs des longueurs d'onde d'excitation et d'émission pour que l'étude soit réalisable, un coefficient d'extinction adapté à la concentration de la sonde utilisée pour l'étude, le rendement quantique qui permet d'avoir un signal important sans perte d'énergie et stable dans le temps, c'est-à-dire possédant une demi-vie assez longue à l'état excité.

De plus il est souhaitable que le fluorophore sélectionné participe le moins possible à des réactions chimiques dans son environnement au cours de la mesure, ce qui conduirait à une perte de ses propriétés de fluorescence, c'est-à-dire une perte du signal.

Les dérivés ayant un squelette benzoxadiazole, appelés aussi benzofurazanes, possèdent les propriétés de fluorescence requises. Les valeurs des longueurs d'ondes utilisées pour l'excitation et l'émission, ainsi que la qualité du phénomène en intensité et stabilité, sont reliés à la structure du composé, et de ses substituants.

Ces dérivés sont connus, les études de fluorescence en fonction de leurs substituants ainsi que leur préparation sont décrits (J. Chem. Soc. Perkin Trans 2, 1998, 2165, J. Chem.Soc, Perkin Trans 2, 1999, 569).

Des dérivés fluorescents benzoxadiazoles pour un usage biologique ont été étudiés (Anal. Chim. Acta, 2005, 550, 173, Bioorg. Med. Chem. Lett. 2003, 13, 1717, Luminscence, 2002, 17, J. Med.Chem. 1999, 42, 3101).

Le cancer est encore une des principaies causes de mortalité du monde occidental. Les moyens de lutte que sont la prévention, la chirurgie, la radiothérapie, l'immunothérapie et la chimiothérapie ne permettent pas encore, dans de nombreux cas, d'éradiquer la maladie.

Les raisons de cet échec reposent en partie sur la difficulté d'identifier la cellule tumorale et de la traiter sélectivement sans trop endommager le tissu sain.

Des composés portant un groupe fluorescent sont décrits dans l'art antérieur pour leur utilisation dans l'étude des cellules cancéreuses, tels que :
- des peptides, substrats de l'acétyltransférase PAT, portant un marqueur fluorescent (NBD) utiles pour l'étude de l'activité de PAT, notamment dans des cellules cancéreuses (Ducker et al. Methods 2006, 40, 166-170) ;
- deux dérivés fluorescents de la choline et de la phosphatidylcholine (NBD-choline et C₆-NBD-PC) permettant l'étude de la distribution et de l'absorption de la choline dans des cellules normales ou cancéreuses (Villa et al. European Journal of Cancer 2005, 41, 1453-1459) ;
- des analogues fluorescents du méthylbenzoprim (MBP) portant un groupe fluorophore DNS, NBD ou MMC utiles comme sonde pour la caractérisation du transport cellulaires, de l'accumulation et de la distribution d'antifolates lipophiles, notamment dans des lignées cellulaires cancéreuses surexprimant Pgp ou MRP (Robson et al. Biochemical Pharmacology 1998, 56, 807-816).

Les molécules hybrides conjuguées benzoxadiazoles - polyamines permettent également de répondre à la problématique de l'identification et du traitement selectif de cellules tumorales.

En effet, la cellule cancéreuse nécessite pour sa prolifération un grand nombre d'éléments biologiques indispensables parmi lesquels sont présents les polyamines (spermine, spermidine, putrescine). Les polyamines sont indispensables à toute cellule en prolifération. En situation normale, la synthèse endogène de polyamine suffit à la cellule, mais dans le cas des cellules cancéreuses, un apport exogène de polyamines à l'aide d'un transport actif est le plus souvent nécessaire. Ainsi, la mise en évidence de ce transport actif permet de détecter les cellules cancéreuses et de suivre leur-progression en terme de croissance et de dissémination. Cependant, le transporteur de polyamine, dans les cellules humaines, n'est, à ce jour, pas identifié au niveau moléculaire. La seule méthode pour démontrer sa présence est donc d'utiliser une sonde reconnue par ce transporteur qui, en s'accumulant dans la cellule, rendra compte de l'activité du transporteur.

Le transporteur de polyamines est un complexe protéique dont la structure n'est pas connue dans les détails.

Ce transporteur joue un rôle d'importation intracellulaire des polyamines naturelles que sont la putrescine, la spermidine, et la spermine.

Ces polyamines sont essentielles au développement et à la croissance cellulaire. Le métabolisme intra cellulaire pourvoit normalement aux besoins de la cellule saine. Ce métabolisme fourni ces polyamines à partir des acides aminés que la cellule possède. L'arginine se transforme en ornithine, l'ornithine subit une décarboxylation pour fournir la putrescine. Cette putrescine (C4) peut s'allonger d'une chaîne en C3 grâce à une adénosine méthionine, suivi de décarboxylation, fournissant la spermidine. Cette dernière peut à nouveau s'allonger d'un motif en C3 grâce aussi à un autre résidu méthionine, fournissant la spermine.

Ces polyamines également peuvent se dégrader par oxydation avec des polyamineoxydases, et ainsi se retransformer en spermidine et putrescine.
Si le mécanisme intracellulaire est perturbé, par un dysfonctionnement quelconque (ex : cellule cancéreuse), le métabolisme intracellulaire ne suffit plus aux besoins de la cellule, ainsi elle va se pourvoir à l'extérieur en polyamines.
C'est ici que le transporteur de polyamine intervient. Il joue un rôle d'alimentation et de régulation.

Dans le cas par exemple du composé de l'exemple 27 de la demande de brevet WO 2005/100363 : le motif structural « épipodophyllotoxine » greffé sur la spermine ne semble pas modifier la reconnaissance du transporteur de polyamines ce qui permet d'internaliser le composé dans la cellule tumorale, qui ultérieurement jouera son rôle d'agent cytotoxique. De la même façon les sondes fluorescentes possédant un motif polyamine seront reconnues par le transporteur de polyamines.

Il existe cependant parmi le panel de lignées cellulaires étudiées en cancérologie, une variabilité dans l'expression du transporteur de polyamines. A l'aide des sondes de diagnostic, il est possible d'identifier par avance (avant traitement antitumoral) les cellules qui seront sensibles au traitement et celles qui ne le seront pas.

Plus la fluorescence intracellulaire sera forte, plus la cellule en question sera sensible au traitement avec un anticancéreux ciblé (par exemple le composé de l'exemple 27 de la demande de brevet WO 2005/100363).

La présente invention se rapporte donc à des composés constitués d'un motif polyamine lié à un motif benzoxadiazole substitué, porteur de la fluorescence qui peuvent être utilisés comme de telles sondes. En effet, la partie polyamine de ces molécules sera reconnue par le système de transport des polyamines et la molécule sera internalisée au sein de la cellule cancéreuse, tandis que le motif porteur de la fluorescence sera détecté par les systèmes d'analyse classiques en milieu biologique, permettant ainsi de sélectionner les tumeurs qui expriment le système de transport des polyamines.

La mise en évidence de ce système de transport des polyamines sur les cellules permet donc de sélectionner les patients porteurs de tumeurs susceptibles d'être soignées par tout composé anticancéreux vectorisé par le système de transport des polyamines, quel que soit son mécanisme d'action.

Ainsi, pourra être révélé dans les tumeurs prélevées par biopsies ou dans les cellules leucémiques prélevées par ponction chez les patients hospitalisés concernés, une fluorescence proportionnelle à l'expression du transporteur de polyamine. Les patients porteurs de ces tumeurs pourront alors être sélectionnés afin d'être traités préférentiellement avec les composés anticancéreux vectorisés vers le système de transport des polyamines, en étant assurés de bien meilleures chances de réponse au traitement.

Le brevet US n° 6,172,261 *et* Anal. Chim. Acta, 1983, 149, 305 décrivent des polyamines liées à des groupes fluorophores. Plus particulièrement B.R. Ware décrit dans Langmuir (1988, 4, 458) un dérivé de spermine avec un groupe benzoxadiazole, dont la formule est donnée ci-après, et son utilisation comme dérivé fluorescent de polyamine dans l'étude de son interaction avec des oligonucléotides.

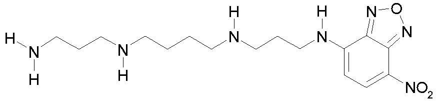

De même, Ohba dans (Tetrahedron, 2007, 63, 37-54) décrit un dérivé de putrescine avec un groupe benzoxadiazole dont la formule est donnée ci-après :

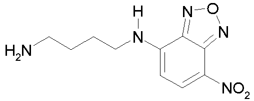

Mais, aucune de ces publications ne mentionne l'utilisation des dérivés de polyamine-benzoxadiazole pour mettre en évidence les cellules tumorales exprimant le système de transport des polyamines.

La présente invention concerne de nouveaux dérivés fluorescents constitués d'un motif polyamine sur lequel est lié un motif porteur de la fluorescence, le benzoxadiazole substitué, leur procédé de préparation et leur utilisation à la mise en évidence du système de transport des polyamines à l'intérieur des cellules. Elle concerne également leur utilisation à la sélection des tumeurs exprimant le système de transport des polyamines ainsi que leur utilisation dans la sélection de patients porteurs de telles tumeurs.

Ces dérivés correspondent à la formule 1 suivante : 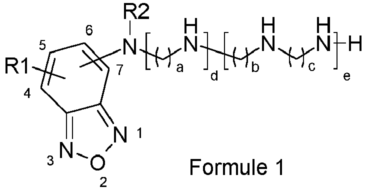 ou un sel pharmaceutiquement acceptable de celui-ci, dans laquelle :
R₁ représente un ou deux groupes NO₂ en position 4 et/ou 6, la chaîne polyamine peut être en position 5, 6 ou 7 ;
R₂ représente un hydrogène, un alkyle en C₁-₆, un benzyle, un groupe perfluoroalkyle ;
les valeurs de a, b, c peuvent être de 2 à 5, indépendamment les unes des autres, et représentent des enchaînements alkylènes séparant les groupes aminés ;
les valeurs de d et e peuvent être indépendamment de 0 ou 1 mais ne peuvent pas représenter en même temps la valeur 0 ;
à la condition que lorsque e = 0, alors R2 ne peut représenter un hydrogène ; et
à l'exception du composé pour lequel R₁ = 4-NO₂, R₂ = H, a = 3, b = 4, c =3 et d = e = 1.

Les composés de la présente invention peuvent être sous forme de sels d'addition minéraux ou organiques.

Dans la présente invention, on entend par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

Par « sel pharmaceutiquement acceptable » d'un composé, on entend désigner dans la présente invention des sels qui sont pharmaceutiquement acceptables, comme défini ici, et qui possèdent l'activité pharmacologique souhaitée du composé parent.

Par « sel d'addition », on entend plus particulièrement, au sens de la présente invention, un sel d'addition d'un acide minéral ou organique.

A titre d'exemple, on peut citer les sels d'addition d'acide formés avec des acides minéraux tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires.

Par « 4-NO₂ », on entend, au sens de la présente invention, un groupe NO₂ (nitro) en position 4.

Par « alkyle », on entend, au sens de la présente invention, une chaîne hydrocarbonée saturée, linéaire ou ramifiée, comportant de 1 à 6 atomes de carbone. En particulier, il peut s'agir d'un groupe méthyle, éthyle, n-propyle, isopropyle, n-butyle, tert-butyle, isobutyle, pentyle ou encore hexyle.
Par « perfluoroalkyle », on entend, au sens de la présente invention, un groupe alkyle tel que défini ci-dessus où tous les atomes d'hydrogènes ont été remplacés par des atomes de fluor.

Avantageusement, R₁ représente un groupe NO₂ en position 4.
Plus particulièrement la présente invention concerne les composés dans lesquels :
- R₁ = 4-NO₂, R₂ = alkyle en C₁₋₆, a = 3, b = 4, c = 3, d = e =1,
- R₁ = 4-NO₂, R₂ = H ou alkyle en C₁₋₆, a = 0, b = 4, c = 3, d = 0, e = 1,
- R₁ = 4-NO₂, R₂ = H ou alkyle en C₁₋₆, a = 0, b = 3, c = 4, d = 0, e = 1, ou
- R₁ = 4-NO₂, R₂ = alkyle en C₁₋₆, a = 4, b = 0, c = 0, d = 1, e = 0.

Encore plus particulièrement l'invention concerne les composés suivants :
- Le N-(3-Aminopropyl)-N'-{3-[méthyl-(7-nitrobenzo[1,2,5]oxadiazol-4-yl)-amino]-propyl}-butane-1,4-diamine correspondant au composé de formule 1 pour lequel R₁ = 4-NO₂, R₂ = Méthyl, a =3, b= 4, c = 3, d = e =1,
- Le N-(3-Aminopropyl)-N'-méthyl-N'-(7-nitrobenzo[1,2,5]oxadiazol-4-yl)-butane-1,4-diamine correspodant au composé de formule 1 pour lequel R₁ = 4-NO₂, R₂ = Méthyl, a = 0, b = 4, c = 3, d = 0, e = 1,
- Le N-(3-Aminopropyl)-N'-(7-nitrobenzo[1,2,5]oxadiazol-4-yl)-butane-1,4-diamine correspondant au composé de formule 1 pour lequel R₁ = 4-NO₂ R₂ = H, a = 0, b = 4, c=3, d = 0, e = 1,
- Le composé de formule 1 pour lequel R₁ = 4-NO₂, R₂ = Méthyl, a = 0, b = 3, c = 4, d = 0, e = 1,
- Le composé de formule 1 pour lequel R₁ = 4-NO₂, R₂=H, a = 0, b = 3, c = 4, d = 0, e = 1, et
- Le composé de formule 1 pour lequel R₁ = 4-NO₂, R₂ = Méthyl, a = 4, b = 0, c = 0, d = 1, e = 0.

L'invention concerne également un procédé de synthèse des composés de formule 1 caractérisé en ce qu'il comprend les étapes a), b) et c) suivantes :
a) Couplage d'un dérivé de benzoxadiazole de formule 2 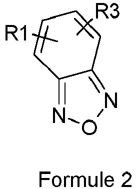 dans laquelle R₁ représente un ou 2 groupes NO₂ en position 4 et/ou 6 ; et avantageusement un groupe NO₂ en position 4 ;
   R₃ représente un substituant halogène mobile en position ortho ou para du groupe R₁,
   avec une polyamine de formule 6, en position 5, 6 ou 7, c'est-à-dire en ortho ou para du groupe R₁, dans laquelle P représente un groupement protecteur des fonctions amine et où a, b, c, d et e ont les mêmes significations que celles données précédemment.

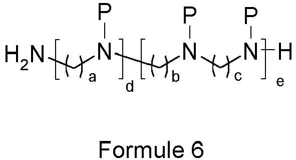

Les groupements protecteurs P peuvent être des groupements BOC (tertiobutyloxycarbonyle) clivables en milieu acide (HCl ou acide trifluoroacétique (TFA))

Au cours de cette réaction de couplage la polyamine réagit avec le benzoxadiazole par substitution de l'halogène par traitement avec une base dans un solvant inerte.

Plus particulièrement, le composé de formule 2 est le 4-nitro-7-chlorobenzoxadiazole.

Plus particulièrement encore, le solvant inerte est l'acétonitrile et la base le carbonate de Cesium.

Les différents benzoxadiazoles sont décrits dans les publications suivantes : J. Chem. Soc. Perkin Trans 2, 1998, 2165, J. Chem.Soc, Perkin Trans 2, 1999, 569. Des exemples de dérivés polyamine, les dérivés spermine protégés par 3 groupes benzyloxycarbonyel ou tertiobutyloxycarbonyle, sont décrits dans la publication : (Tet. Let. 1998, 39, 439).
b) Le composé issu de la réaction de couplage a) est alkylé par un halogénure d'alkyle en présence d'une base dans un solvant inerte. On utilisera préférentiellement le THF (tétrahydrofurane) comme solvant.
De manière préférentielle, on utilisera comme base l'hydrure de sodium ou un carbonate alcalin.

De manière préférentielle également, on utilisera le THF comme solvant.
c) Le composé issu de l'étape b) est ensuite déprotégé en milieu acide à température ambiante pour obtenir les composés de formule 1.

De manière préférentielle cette étape de déprotection se fait dans l'acide chlorhydrique ou dans l'acide trifluoroacétique.

La présente invention concerne également l'utilisation des composés de formule 1 suivante : 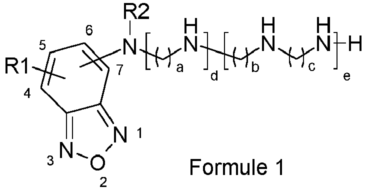 ou un sel pharmaceutiquement acceptable de celui-ci,
dans laquelle :
R₁ représente un ou deux groupes NO₂ en position 4 et/ou 6, la chaîne polyamine peut être en position 5, 6 ou 7 ;
R₂ représente un hydrogène, un alkyle en C₁-₆, un benzyle, un groupe perfluoroalkyle ;
les valeurs de a, b, c peuvent être de 2 à 5, indépendamment les unes des autres, et représentent des enchaînements alkylènes séparant les groupes aminés ;
les valeurs de d et e peuvent être indépendamment de 0 ou 1 mais ne peuvent pas représenter en même temps la valeur 0 ;
comme sonde fluorescente de diagnostic pour la mise en évidence des tumeurs exprimant le système de transport des polyamines.

Les composés de la présente invention peuvent être sous forme de sels d'addition minéraux ou organiques.
Avantageusement, R₂ ne représentera pas un hydrogène lorsque e = 0.

Avantageusement, R₁ représente un groupe NO₂ en position 4.
Plus particulièrement, les composés de formule 1 utiles comme sonde fluorescente de diagnostic concernent les composés dans lesquels :
- R₁ = 4-NO₂, R₂ = alkyle en C₁₋₆, a = 3, b = 4, c = 3, d = e =1,
- R₁ = 4-NO₂, R₂ = H ou alkyle en C₁₋₆, a = 0, b = 4, c = 3, d = 0, e = 1,
- R₁ = 4-NO₂, R₂ = H ou alkyle en C₁₋₆, a = 0, b = 3, c = 4, d = 0, e = 1, ou
- R₁ = 4-NO₂, R₂ = alkyle en C₁₋₆, a = 4, b = 0, c = 0, d = 1, e = 0.
Encore plus particulièrement, les composés de formule 1 concernent les composés suivants :
- Le N-(3-Aminopropyl)-N'-{3-[méthyl-(7-nitrobenzo[1,2,5]oxadiazol-4-yl)-amino]-propyl}-butane-1,4-diamine correspondant au composé de formule 1 pour lequel R₁ = 4-NO₂, R₂ = Méthyl, a =3, b= 4, c = 3, d = e =1,
- Le N-(3-Aminopropyl)-N'-méthyl-N'-(7-nitrobenzo[1,2,5]oxadiazol-4-yl)-butane-1,4-diamine correspodant au composé de formule 1 pour lequel R₁ = 4-NO₂, R₂ = Méthyl, a = 0, b = 4, c = 3, d = 0, e = 1,
- Le N-(3-Aminopropyl)-N'-(7-nitrobenzo[1,2,5]oxadiazol-4-yl)-butane-1,4-diamine correspondant au composé de formule 1 pour lequel R₁ = 4-NO₂ R₂ = H, a = 0, b = 4, c = 3, d = 0, e = 1,
- Le composé de formule 1 pour lequel R₁ = 4-NO₂, R₂ = Méthyl, a = 0, b = 3, c = 4, d = 0, e = 1,
- Le au composé de formule 1 pour lequel R₁ = 4-NO₂, R₂ = H, a = 0, b = 3, c = 4, d = 0, e = 1, et
- Le composé de formule 1 pour lequel R₁ = 4-NO₂, R₂ = Méthyl, a = 4, b = 0, c = 0, d = 1, e = 0.

Les exemples suivants décrivent les différents stades pour la préparation des composés de l'invention et ne sont en aucune manière limitatifs.

### Exemple 1 : Synthèse du N-(3-aminopropyl)-N'-{3-[méthyl-(7-nitrobenzo[1,2,5]oxadiazol-4-yl)-amino]-propyl}-butane-1,4-diamine.

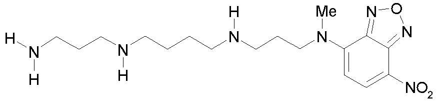

La synthèse de ce composé de formule 1 est réalisée selon le schéma suivant :

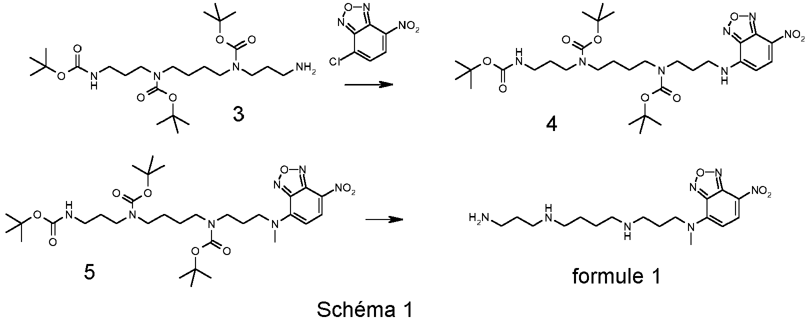

### Stade 1 : Préparation de la triBOC spermine de formule 3 (Bioorg. Med. Chem. 2002, 10, 2397).

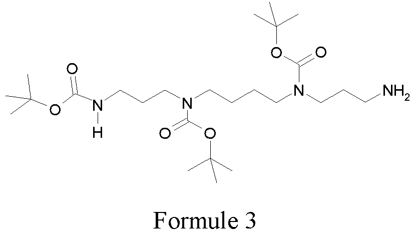

40 g de spermine sont placés en solution dans 300 mL de CH₂Cl₂, et refroidis à 0°C. On ajoute goutte à goutte 23,8 mL de trifluoroacétate d'éthyle en solution dans 50 mL de CH₂Cl₂. La solution sous agitation est laissée 1 heure ensuite à température ambiante. Le résidu obtenu est repris dans 600 mL de THF, on y introduit 140,28 mL de triéthylamine. Une solution de 174,48 g de BOC₂O dans 200 mL de THF est alors introduite en maintenant la température à 20°C. L'agitation à température ambiante est maintenue encore trois heures, puis le milieu réactionnel est jeté sur de l'eau et extrait par l'éther isopropylique. Après décantation, séchage sur sulfate de sodium, filtration et évaporation, la solution organique est évaporée. Le résidu obtenu est alors repris dans 900 mL d'un mélange MeOH/H₂O 8/2. On ajoute 192,6 g de carbonate de Césium et on porte le tout à reflux pendant trois heures. Après évaporation du méthanol sous pression réduite, on ajoute de l'eau et on extrait par de l'éther isopropylique. On décante la phase organique, puis on introduit dans cette phase organique de l'acide chlorhydrique 0,5 N. On laisse décanter. Il apparaît 3 phases. La phase du milieu sous forme huileuse contient le produit attendu sous forme de chlorhydrate. On décante cette phase, puis on la reprend par du CH₂Cl₂, et on sèche sur sulfate de sodium. Après filtration et évaporation, on obtient 54 g du chlorhydrate de la triBOC spermine sous forme d'une huile incolore utilisée directement dans l'étape suivante (Rdt = 50%). CCM SiO₂ : CH₂Cl₂-MeOH-NH₄OH (90-10-10) Rf = 0,5.

Stade 2 : Condensation avec le fluorophore.

5,39 g de l'huile obtenue au stade 1 sont mis en solution dans 100 mL d'acétonitrile, de 6,42 g de carbonate de Césium et de 2 g de 4-chloro-7-nitrobenzo[1,2,5]oxadiazole sous agitation. Le mélange est porté au reflux pendant une heure. Le milieu réactionnel est fixé sur de la silice et le solvant évaporé. Le résidu solide est placé en tête de colonne de flash chromatographie, et éluée avec un gradient partant de l'heptane pur au mélange heptane-AcOEt (50-50). On obtient alors 4,4 g (66%) d'une huile rouge. CCM : Si02 Heptane/AcOEt (50-50) Rf = 0,3. SM (APCI) m/z = 666,3 (MH+) ; ¹H RMN (CDCl₃) : 8,47(d, 1H, J = 8Hz, H₅), 7,96(m, 1H, NH), 6,17(d, 1H, J = 8Hz, H₆), 3,53(m, 2H, NHCH₂), 3,38(m, 2H, CH₂NH), 3,10-3,21(m, 8H, NCH₂), 1,92(m, 2H, CH₂), 1,66(m, 2H, CH₂), 1,43-1.51(m, 31H, CH₂, t-Bu).

Stade 3 : Alkylation en R₂. Préparation de l'ester tertiobutylique de l'acide (3-{t-butoxycarbonyl-[4-(t-butoxycarbonyl-{3-[méthyl-(7-nitrobenzo[1,2,5]oxadiazol-4-yl)-amino]-propyl}-amino)-butyl]-amino}-propyl)-carbamique de formule 5.

Le composé obtenu au stade précédent (4,4 g) est dissous dans 150 mL d'acétonitrile, en présence de 4 g de carbonate de Césium et 2,05 mL d'iodure de méthyle. Le milieu réactionnel est agité à température ordinaire pendant quatre heures. Le milieu est fixé sur silice par évaporation du solvant puis chromatographié sur une colonne flash de diamètre 50 mm. Par élution en gradient à partir de l'heptane pur au mélange heptane -AcOEt (50-50), on obtient 4,2 g d'une huile rouge (Rdt = 93%). CCM : Heptane-AcOEt (30-70) Rf = 0,4. SM (ESI) m/z = 680,4 (MH+) ; ¹H RMN (DMSO) : 8,48(d, 1H, J = 8Hz, H₅), 6,40(d, 1H, J = 8Hz, H₆), 3,47(m, 2H, NHCH₂), 3,33(s, 3H, NCH₃), 3,24(m, 2H, CH₂NH), 3,15(m, 2H, NCH₂), 3,08(m, 4H, NCH₂), 2,88(m, 2H, NCH₂), 1,91(m, 2H, CH₂), 1,54(m, 2H, CH₂), 1,36(m, 31H, CH₂, t-Bu). Stade 4 : 4,2 g du composé du stade précédent sont dissous dans 20 mL d'une solution 4M d'HCl dans le dioxane et maintenus trois heures sous agitation à température ordinaire. Le précipité orange du chlorhydrate est alors filtré rincé par du méthanol puis par de l'éther éthylique. Après séchage sous pression réduite, on obtient 2,8 g (92%) de sel du N-(3-aminopropyl)-N'-{3-[méthyl-(7-nitrobenzo[1,2,5]oxadiazol-4-yl)-amino]-propyl}-butane-1,4-diamine de formule 1. F=260°C. SM (ESI) m/z = 380,2 (MH+) ; ¹H RMN (D₂O) : 8,50(d, 1H, J = 8Hz, H₅), 6,41(d, 1H, J = 8Hz, H₆), 4,27(m, 2H, NHCH₂), 3,54(s, 3H, NCH₃), 3,11-3,27(m, 10H, CH₂NH), 2,26(m, 2H, CH₂), 2,11(m, 2H, CH₂), 1,82(m, 4H, CH₂).

De la même façon, les composés de formule 1 où d = 0, e = 1 et b = 3, c = 4 ou bien b = 4 et c = 3, peuvent être préparés selon la même méthodologie en utilisant la spermidine diprotégèe. Les différentes méthodes utilisées pour préparer ces polyamines protégées de formule 6 (P = benzyloxycarbonyle ou bien tertiobutyloxycarbonyle) sont soit décrites dans la demande de brevet WO 2005/100363, soit indiquées dans les publications mentionnées.

### Exemple 2 : Synthèse de la N-1-(7-Nitrobenzo[1,2,5]oxadiazol-4-yl]butane-1,4-diamine

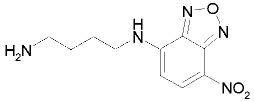

Ce composé est décrit dans la publication de Ohba & al suivante : (Tet. 2007, 63, 3754).

Il est synthétisé selon la présente invention par le procédé suivant :
Stade 1 : Porter à reflux 30 minutes, dans 5 mL d'acétonitrile, un mélange constitué de 200 mg de 4-chloro-7-nitrobenzo[1,2,5]oxadiazole et de 190 mg d'ester tertiobutylique de l'acide (4-aminobutyl)-carbamique, et 320 mg de carbonate de Césium. Après refroidissement le milieu est versé dans l'eau et extrait avec de l'acétate d'éthyle. Décanter et sécher la phase organique sur sulfate de sodium. Filtrer et évaporer sous pression réduite. Une chromatographie flash sur silice éluée avec un gradient de l'heptane pur à l'acétate d'éthyle pur en 30 minutes, permet d'isoler après évaporation 280 mg (rdt 80%) de l'ester tertiobutylique de l'acide [4-(7-nitrobenzo[1,2,5]oxadiazol-4-ylamino)-butyl]-carbamique sous forme d'une huile. CCM SiO₂ : Heptane-AcOEt (30-70), Rf = 0,5. SM (ESI) m/z = 352,1 (MH+) ; ¹H RMN (DMSO) 9,55(s, 1H, NH), 8,50(d, 1H, J = 8Hz, H₅), 6,83(m, 1H, NH), 6,42(d, 1H, J = 8Hz, H₆), 3,47(m, 2H, NHCH₂), 2,96(m, 2H, CH₂NH), 1,66(m, 2H, CH₂), 1,48(m, 2H, CH₂), 1,36(s, 9H, t-Bu).
Stade 2 : 280 mg du carbamate obtenu au stade précédent sont mis en solution dans 15 mL de chlorure de méthylène et 2 mL d'acide trifluoroacétique, puis agités pendant 15 minutes à température ambiante. Après évaporation, le résidu huileux obtenu est cristallisé dans l'éther isopropylique. On isole 150 mg (rdt 51%) de trifluoroacétate de N-1-(7-nitrobenzo[1,2,5]oxadiazol-4-yl]butane-1,4-diamine. CCM SiO₂ : CH₂C₁₂-MeOH-NH₄OH (90/10/1), Rf = 0,7. SM (ESI) m/z = 252,1 (MH+) ; ¹H RMN (DMSO) 9,54(s, 1H, NH), 8,53(d, 1H, J = 8Hz, H₅), 7,70(s, 2H, NH₂), 6,44(d, 1H,
   J = 8Hz, H₆), 3,50(m, 2H, NHCH₂), 2,84(m, 2H, CH₂NH), 1,59-1,76(m, 4H, CH₂CH₂).

### Exemple 3 (comparatif) : Synthèse de la N-(3-Aminopropyl)-N'-[3-(7-méthylbenzo[1,2,5]oxadiazol-4-ylamino)-propyl]-butane-1-4-diamine.

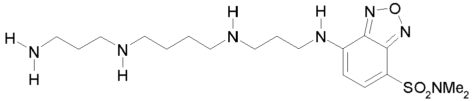

Stade 1 : 250 mg de triBOC spermine obtenue au stade 1 de l'exemple 1 sont mis à réagir avec 100 mg de diméthylamide de l'acide 4-fluorobenzo[1,2,5]oxadiazole-4-sulfonique en présence de 0,17 mL de triéthylamine dans 10 mL d'acétonitrile sous agitation à température ordinaire pendant 30 minutes. Le milieu réactionnel est jeté sur de l'eau acidifiée avec HCl et extrait avec de l'acétate d'éthyle. Après décantation et lavage de la phase organique par de l'eau saturée de NaCl, la solution est séchée sur Na₂SO₄, filtrée et évaporée. On obtient 250 mg d'une huile jaune (Rdt = 84%). SM (ESI) m/z = 728,5 (MH+) ; ¹H RMN (DMSO) : 7,83(d, 1H, J = 8Hz, H₅), 6,30(d, 1H, J = 8Hz, H₆), 3,35(m, 2H, NHCH₂), 2,68(s, 6H, NMe₂), 3,23(m, 2H, CH₂N), 3,14(m, 6H, CH₂N), 2,87(m, 2H, NCH₂), 1,86(m, 2H, CH₂), 1,55(m, 2H, CH₂), 1,36(m, 31H, CH₂, t-Bu).
Stade 2 : 250 mg du composé protégé précédemment obtenu sont agités à température ambiante pendant 30 minutes dans 2 mL d'acide trifluoroacétique et 10 mL de CH₂Cl₂. Après évaporation, on cristallise avec un rendement quantitatif 260 mg de trifluoroacétate de N-(3-aminopropyl)-N'-[3-(7-méthylbenzo[1,2,5]oxadiazol-4-ylamino)-propyl]-butane-1-4-diamine dans l'éther isopropylique, avec des traces d'acétone. SM (ESI) m/z = 428,3 (MH+) ; ¹H RMN (DMSO) : 7,84(d, 1H, J = 8Hz, H₅), 6,37(d, 1H, J = 8Hz, H₆), 2,85-3,05(m, 12H, NHCH₂), 2,70(s, 6H, NMe₂), 2,00(m, 2H, CH₂), 1,90(m, 2H, CH₂), 1,62(s, 4H, CH₂).

### Exemple 4 : Etude pharmacologique

### 1. Sélectivité des sondes vis-à-vis des cellules tumorales.

Les cellules issues de biopsies sont dissociées mécaniquement en présence ou non de trypsine selon la constitution du tissu considéré. Les cellules hématopoiétiques sont séparées et isolées selon les méthodes de préparation classiques des cellules sanguines. Une fois isolées dans un temps assez court pour assurer la viabilité des cellules étudiées, les cellules sont mises en présence de la sonde telle que synthétisée par exemple dans les exemples 1 à 3 et laissées incubées dans le milieu de culture recommandé pour le tissu considéré en présence d'aminoguanidine. L'aminoguanidine permet d'éviter une dénaturation *in vitro* des polyamines. On procèdera en absence de sérum (trop riche en polyamine et donc source d'erreur de mesure). Une incubation de une à quatre heures est suffisante et permet une détection de la fluorescence par immunochimie ou par cytométrie en flux en choisissant les longueurs d'onde d'excitation et d'émission compatibles avec le substituant de la sonde fluorescente. Cette analyse est réalisée par un cytomètre en flux ou par histochimie selon une méthodologie standard en excitant, dans l'étude ci-dessous la sonde obtenue à l'exemple 1, le N-(3-aminopropyl)-N'-{3-[méthyl-(7-nitrobenzo[1,2,5]oxadiazol-4-yl)-amino]-propyl}-butane-1,4-diamine par une source de 488nm et en mesurant l'émission de fluorescence à 525nm. Dans ce même exemple, des cellules A549 dérivées d'une tumeur humaine d'un cancer du poumon non à petites cellules ont été testées en comparaison de cellules MRC-5 issues d'un poumon sain et disponibles commercialement auprès de banques de lignées cellulaires.

### Résultats :

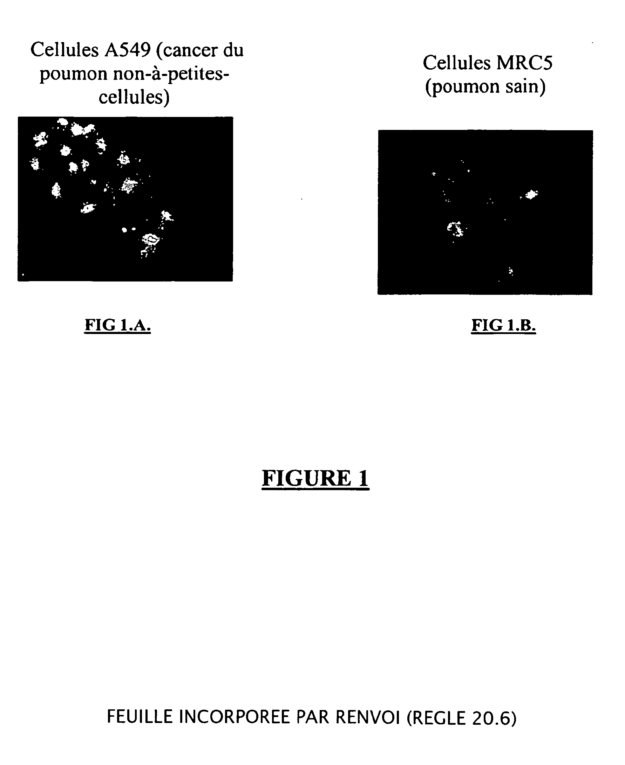

La figure 1 annexée donne les images obtenues par un microscope à fluorescence standard des cellules cultivées sur lame. Elle montre une incorporation bien supérieure de la sonde de l'exemple 1 dans les cellules tumorales (Figure 1A) que dans les cellules issues de tissus sain (Figure 1B). Ce différentiel a également été confirmé par cytométrie en flux.

Ce résultat confirme l'intérêt d'utiliser les composés de la présente invention comme sonde fluorescente de diagnostic permettant de sélectionner les tumeurs éligibles pour un traitement avec un composé anticancéreux vectorisé par le transporteur de polyamines.

### 2. Prédictivité de la réponse aux agents anticancéreux vectorisés vers le système de transport des polyamines.

Afin de comparer l'incorporation des sondes telles que décrites ci-dessus et ainsi de démontrer le caractère prédictif de la réponse aux agents vectorisés vers le système de transport des polyamines, une mesure de la cytotoxicité par une méthode standard (ici la mesure de l'ATP résiduel à l'aide du kit ATPlite de la société Perkin-Elmer) a été réalisée. On utilisera un composé anticancéreux dérivé de l'épipodophyllotoxine dont la pénétration intracellulaire dépend du transporteur de polyamine, tel le composé décrit dans l'exemple 27 de la demande de brevet WO 2005/100363 et à titre de référence, l'étoposide, une épipodophyllotoxine non vectorisée. Ces 2 composés ont le même mécanisme d'action sur les cellules cancéreuses : ce sont tous les 2 des inhibiteurs de la topoisomérase 2.

Un panel de lignées leucémiques a été testé afin de rendre compte de l'incorporation de sonde, ici celle décrite à l'exemple 1, en regard de la sensibilité à un agent vectorisé tel que le composé de l'exemple 27 de la demande de brevet WO 2005/100363 et en comparaison de l'étoposide non vectorisé. Après 72 heures d'incubation avec le composé de l'exemple 27 de la demande de brevet WO 2005/100363 ou l'étoposide, la cytotoxicité est évaluée à l'aide du kit ATPlite. Les cellules de même lignée ont également été incubées en présence de la sonde décrite à l'exemple 1, et ont été analysées par cytométrie en flux. L'intensité de fluorescence mesurée à 525nm a été suivie de 0 à 1 heure 30 minutes et la pente de la variation d'intensité a été calculée.

### Résultats :

Dans un premier temps la comparaison de la cytotoxicité du composé de l'exemple 27 de la demande de brevet WO 2005/100363 et de l'étoposide de mode d'action similaire, a permis, par l'établissement d'un ratio des IC₅₀, d'identifier les leucémies pour lesquelles la vectorisation avait un rôle prépondérant dans l'efficacité du composé.

Pour l'ensemble des lignées testées, la capacité d'incorporer la sonde fluorescente, caractérisée par la fluorescence mesurée par cytométrie de flux est également mesurée.

Ces résultats sont donnés dans le tableau 1 suivant :

**Tableau 1**

| Lignée cellulaire | Cytotoxicité de l'Etoposide IC₅₀ (µM) | Cytotoxicité du composé de l'exemple 27 de WO 2005/100363 IC₅₀ (µM) | Ratio d' IC₅₀ Etoposide/composé de l'exemple 27 de WO 2005/100363 | Activité de transport des polyamines* |
|---|---|---|---|---|
| CEM | 0,06 | 0,003 | 20 | 421 |
| BL-41 | 0,4 | 0,009 | 44 | 164 |
| BLUE-1 | 1 | 0,1 | 10 | 58 |
| U-937 | 0,4 | 0,04 | 10 | 33 |
| KG-1 | 5 | 5 | 1 | 17 |

| | | | | |
|---|---|---|---|---|
| * Unité arbitraire d'intensité de fluorescence : (intensité des cellules en présence de sonde) - (intensité des cellules en l'absence de sonde). | | | | |

Il apparaît clairement que l'intensité de fluorescence est la plus forte dans les cellules pour laquelle la vectorisation est également importante.

En conclusion, le fait de mesurer l'incorporation de la sonde permet de prédire une meilleure efficacité et une meilleure sélectivité antitumorale d'un composé anticancéreux tel que le composé de l'exemple 27 de la demande de brevet WO 2005/100363. Ces résultats démontrent que le suivi de l'incorporation de la sonde permet d'identifier des cellules leucémiques disposant d'un fort transport actif des polyamines, et par conséquent susceptibles d'être traitées efficacement par les composés anticancéreux vectorisés par le transporteur de polyamines.

Le différentiel obtenu par cytométrie en flux est comparable à celui visualisé par histochimie. Il est possible de réaliser un test plus simple en ne mesurant la fluorescence qu'à un temps donné. Ceci a été vérifié en réalisant cette mesure de fluorescence après 1 heure 30 minutes d'incubation seulement. Ceci peut présenter un avantage dans un cadre hospitalier pour apporter une réponse rapide à la sélection de cellules répondeuses.

Par extension, de telles mesures peuvent s'appliquer à des blastes de patients prélevées par prise de sang ou ponction de moelle au cours des analyses de routine. La mesure de l'intensité de fluorescence sera réalisée par une procédure de routine par cytométrie de flux ou histocytochimie dans tous les hôpitaux disposant d'une plate-forme de cytologie et/ou d'anatomopathologie.

## Revendications

1. Utilisation d'un composé de formule générale 1 : 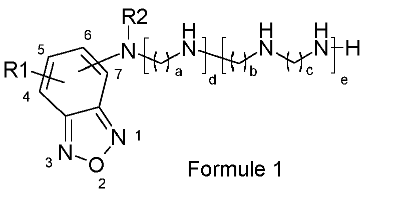 ou un sel pharmaceutiquement acceptable de celui-ci,
dans laquelle :
R₁ représente un ou deux groupes NO₂ en position 4 et/ou 6, ou bien un groupe SO₂Ph, SO₂NMe₂, SO₂NH₂, SO₃H ;
la chaîne polyamine peut être en position 5, 6 ou 7 ;
R₂ représente un hydrogène, un alkyle en C₁-₆, un benzyle, un groupe perfluoroalkyle ;
les valeurs de a, b, c peuvent être de 2 à 5, indépendamment les unes des autres, et représentent des enchaînements alkylènes séparant les groupes aminés ;
les valeurs de d et e peuvent être indépendamment de 0 ou 1 mais ne peuvent pas représenter en même temps la valeur 0 ;
comme sonde fluorescente de diagnostic pour la mise en évidence des tumeurs exprimant le système de transport des polyamines.

2. Composé de formule générale 1 : 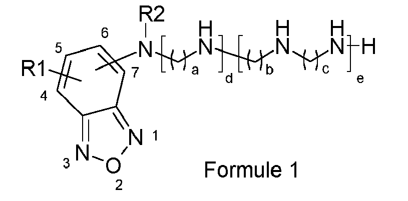 ou un sel pharmaceutiquement acceptable de celui-ci :
dans laquelle :
R₁ représente un ou deux groupes NO₂ en position 4 et/ou 6, ou bien un groupe SO₂Ph, SO₂NMe₂, SO₂NH₂, SO₃H ;
la chaîne polyamine peut être en position 5, 6 ou 7 ;
R₂ représente un hydrogène, un alkyle en C₁-₆, un benzyle, un groupe perfluoroalkyle ;
les valeurs de a, b, c peuvent être de 2 à 5, indépendamment les unes des autres, et représentent des enchaînements alkylènes séparant les groupes aminés ;
les valeurs de d et e peuvent être indépendamment de 0 ou 1 mais ne peuvent pas représenter en même temps la valeur 0 ;
à la condition que lorsque e = 0, alors R2 ne peut représenter un hydrogène ; et
à l'exception du composé pour lequel R₁ = 4-NO₂, R₂ = H, a = 3, b = 4, c =3 et d = e = 1.

3. Composé selon la revendication 2 dans lequel R₁ = 4-NO₂, R₂ = alkyle en C₁₋₆, a = 3, b = 4, c = 3, d = e = 1.

4. Composé selon la revendication 2 dans lequel R₁ = 4-NO₂, R₂ = H ou alkyle en C_{1-6,} a = 0, b = 4, c=3, d = 0, e = 1.

5. Composé selon la revendication 2 dans lequel R₁ = 4-NO₂, R₂ = H ou alkyle en C₁₋₆, a = 0, b = 3, c = 4, d = 0, e = 1.

6. Composé selon la revendication 2 dans lequel R₁ = 4-NO₂, R₂ = alkyle en C₁₆, a = 4, b = 0, c = 0, d = 1, e = 0.

7. Composé selon la revendication 2, le N-(3-Aminopropyl)-N'-{3-[méthyl-(7-nitrobenzo[1,2,5]oxadiazol-4-yl)-amino]-propyl}-butane-1,4-diamine, dans lequel :
R₁ = 4-NO₂, R₂ = Méthyl, a =3, b= 4, c = 3, d = e =1.

8. Composé selon la revendication 2, le N-(3-Aminopropyl)-N'-méthyl-N'-(7-nitrobenzo[1,2,5]oxadiazol-4-yl)-butane-1,4-diamine, dans lequel : R₁ = 4-NO₂, R₂ = Méthyl, a = 0, b = 4, c = 3, d = 0, e = 1.

9. Composé selon la revendication 2, le N-(3-Aminopropyl)-N'-(7-nitrobenzo[1,2,5]oxadiazol-4-yl)-butane-1,4-diamine, dans lequel : R₁ = 4-NO₂, R₂ = H, a = 0, b = 4, c = 3, d = 0, e = 1.

10. Composé selon la revendication 2, dans lequel : R₁ = 4-NO₂, R₂ = Méthyl, a = 0, b = 3, c = 4, d = 0, e = 1.

11. Composé selon la revendication 2, dans lequel : R₁ = 4-NO₂, R₂ = H, a = 0, b = 3, c = 4, d = 0, e = 1.

12. Composé selon la revendication 2, dans lequel : R₁ = 4-NO₂, R₂ = Méthyl, a = 4, b = 0, c = 0, d = 1, e = 0.

13. Procédé de synthèse d'un composé de formule 1 tel que défini à la revendication 1, **caractérisé en ce qu'**il comprend les étapes suivantes:
a) Couplage d'un dérivé de benzoxadiazole de formule 2 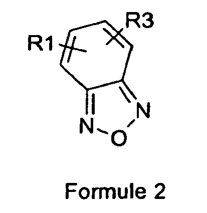 dans laquelle R₁ représente un ou 2 groupes nitro en position 4 et/ou 6 ;
R₃ représente un substituant halogène mobile en position 7,
avec une polyamine de formule 6 dans laquelle P représente un groupement protecteur des fonctions amine et où a, b, c sont compris entre 2 et 5, indépendamment les uns des autres, et où d et e peuvent être indépendamment 0 ou 1 ; 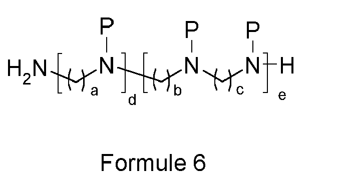
b) Alkylation du composé issu de la réaction de couplage a) est alkylé par un halogénure d'alkyle en présence d'une base dans un solvant inerte ; et
c) Déprotection du composé issu de l'étape b) en milieu acide à température ambiante pour obtenir le composé de formule 1.

## Patentansprüche

1. Verwendung einer Verbindung der allgemeinen Formel 1: 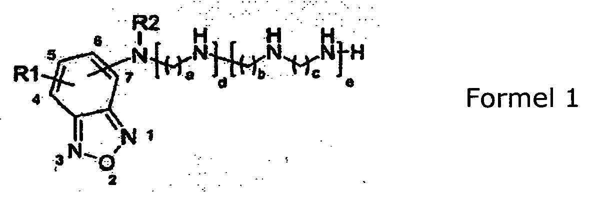 oder eines pharmazeutisch akzeptablen Salzes davon, worin:
R₁ eine oder zwei NO₂-Gruppen an Position 4 und/oder 6 darstellt;
die Polyamin-Kette an Position 5, 6 oder 7 vorliegen kann;
R₂ einen Wasserstoff, ein C₁₋₆-Alkyl, ein Benzyl, eine Perfluoralkyl-Gruppe darstellt;
die Werte von a, b, c unabhängig voneinander 2 bis 5 betragen können und Alkylen-Verbindungen darstellen, welche die Amingruppen voneinander trennen;
die Werte von d und e unabhängig 0 oder 1 sein können, aber nicht gleichzeitig den Wert 0 haben können;
als fluoreszierende Sonde für die Diagnostik zum Nachweis von Tumoren, welche das Polyamin-Transport-System exprimieren.

2. Verbindung der allgemeinen Formel 1: 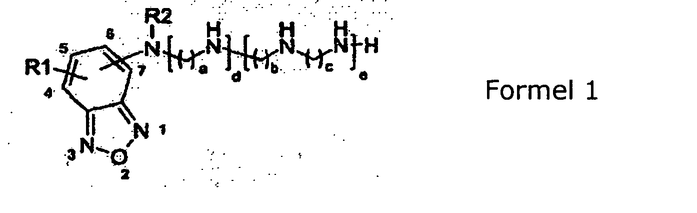 oder ein pharmazeutisch akzeptables Salz davon, worin:
R₁ eine oder zwei NO₂-Gruppen an Position 4 und/oder 6 darstellt;
die Polyamin-Kette an Position 5, 6 oder 7 vorliegen kann;
R₂ einen Wasserstoff, ein C₁₋₆-Alkyl, ein Benzyl, eine Perfluoralkyl-Gruppe darstellt;
die Werte von a, b, c unabhängig voneinander 2 bis 5 betragen können und Alkylen-Verbindungen darstellen, welche die Amingruppen voneinander trennen;
die Werte von d und e unabhängig 0 oder 1 sein können, aber nicht gleichzeitig den Wert 0 haben können;
unter der Bedingung, dass, wenn e = 0, R₂ dann keinen Wasserstoff darstellen kann;
mit Ausnahme der Verbindung, bei der R₁ = 4-NO₂, R₂ = H, a = 3, b = 4, c = 3 und d = e = 1.

3. Verbindung gemäß Anspruch 2, worin R₁ = 4-NO₂, R₂ = C₁₋₆-Alkyl, a = 3, b = 4, c = 3, d = e = 1.

4. Verbindung gemäß Anspruch 2, worin R₁ = 4-NO₂, R₂ = H oder C₁₋₆-Alkyl, a = 0, b = 4, c = 3, d = 0, e = 1.

5. Verbindung gemäß Anspruch 2, worin R₁ = 4-NO₂, R₂ = H oder C₁₋₆-Alkyl, a = 0, b = 3, c = 4, d = 0, e = 1.

6. Verbindung gemäß Anspruch 2, worin R₁ = 4-NO₂, R₂ = C₁₋₆-Alkyl, a = 4, b = 0, c = 0, d = 1, e = 0.

7. Verbindung gemäß Anspruch 2, N-(3-Aminopropyl)-N'-{3-[methyl-(7-nitrobenzo[1,2,5]oxadiazol-4-yl)amino]propyl}-butan-1,4-diamin, worin:
R₁ = 4-NO₂, R₂ = Methyl, a = 3, b = 4, c = 3, d = e = 1.

8. Verbindung gemäß Anspruch 2, N-(3-Aminopropyl)-N'-methyl-N'-(7-nitrobenzo[1,2,5]oxadiazol-4-yl)-butan-1,4-diamin, worin:
R₁ = 4-NO₂, R₂ = Methyl, a = 0, b = 4, c = 3, d = 0, e = 1.

9. Verbindung gemäß Anspruch 2, N-(3-Aminopropyl)-N'-(7-nitrobenzo[1,2,5]oxadiazol-4-yl)-butan-1,4-diamin, worin:
R₁ = 4-NO₂, R₂ = H, a = 0, b = 4, c = 3, d = 0, e = 1.

10. Verbindung gemäß Anspruch 2, worin:
R₁ = 4-NO₂, R₂ = Methyl, a = 0, b = 3, c = 4, d = 0, e = 1.

11. Verbindung gemäß Anspruch 2, worin:
R₁ = 4-NO₂, R₂ = H, a = 0, b = 3, c = 4, d = 0, e = 1.

12. Verbindung gemäß Anspruch 2, worin:
R₁ = 4-NO₂, R₂ = Methyl, a = 4, b = 0, c = 0, d = 1, e = 0.

13. Verfahren zur Herstellung einer Verbindung der Formel 1, wie in Anspruch 1 definiert, **dadurch gekennzeichnet, dass** es die folgenden Schritte umfasst:
a) Kopplung eines Benzoxadiazol-Derivats der Formel 2 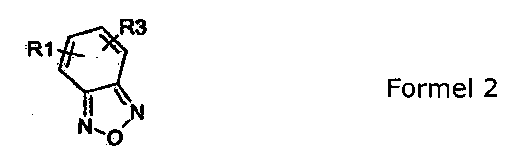 worin R₁ ein oder zwei Nitrogruppen an Position 4 und/oder 6 darstellt;
R₃ einen beweglichen Halogen-Substituenten an Position 7 darstellt,
mit einem Polyamin der Formel 6, worin P eine Schutzgruppe der Aminfunktionen darstellt, und worin a, b, c unabhängig voneinander 2 bis 5 betragen, und worin d und e unabhängig 0 oder 1 sein können; 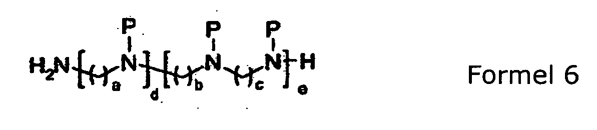
b) Alkylierung der aus der Kopplungsreaktion a) hervorgegangenen Verbindung durch ein Alkylhalogenid in Gegenwart einer Base in einem inerten Lösungsmittel; und
c) Entschützen der aus Schritt b) hervorgegangenen Verbindung in saurem Milieu bei Umgebungstemperatur zum Erhalt der Verbindung der Formel 1.

## Claims

1. Use of a compound of general formula 1: 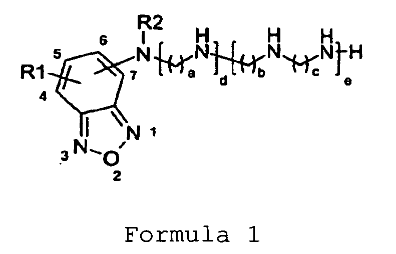 or a pharmaceutically acceptable salt thereof,
in which:
R₁ represents one or two NO₂ groups at position 4 and/or 6;
the polyamine chain can be at position 5, 6 or 7;
R₂ represents a hydrogen, a C₁-₆ alkyl, a benzyl, a perfluoroalkyl group;
the values of a, b, c can be 2 to 5, independently of each other, and represent alkylene chains separating the amino groups;
the values of d and e may be 0 or 1 independently but may not represent the value 0 at the same time;
as fluorescent diagnostic probe to detect tumors expressing the polyamine transport system.

2. Compound of general formula 1: 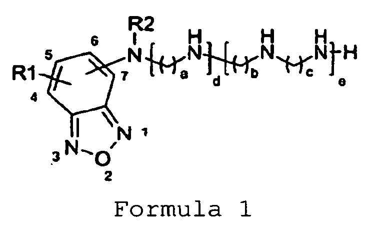 or a pharmaceutically acceptable salt thereof,
in which:
R₁ represents one or two NO₂ groups at position 4 and/or 6;
the polyamine chain can be at position 5, 6 or 7;
R₂ represents a hydrogen, a C₁-₆ alkyl, a benzyl, a perfluoroalkyl group;
the values of a, b, c can be 2 to 5, independently of each other, and represent alkylene chains separating the amino groups;
the values of d and e may be 0 or 1 independently but may not represent the value 0 at the same time;
provided that when e = 0, R₂ may not represent a hydrogen;
with the exception of the compound for which R₁ = 4-NO₂, R₂ = H, a = 3, b = 4, c = 3 and d = e = 1.

3. Compound according to claim 2, in which R₁ = 4-NO₂, R₂ = C₁₋₆ alkyl, a = 3, b = 4, c = 3, d = e = 1.

4. Compound according to claim 2, in which R₁ = 4-NO₂, R₂ = H or C₁₋₆ alkyl, a = 0, b = 4, c = 3, d = 0, e = 1.

5. Compound according to claim 2, in which R₁ = 4-NO₂, R₂ = H or C₁₋₆ alkyl, a = 0, b = 3, c = 4, d = 0, e = 1.

6. Compound according to claim 2, in which R₁ = 4-NO₂, R₂ = C₁₋₆ alkyl, a = 4, b = 0, c = 0, d = 1, e = 0.

7. Compound according to claim 2, N-(3-aminopropyl)-N'-{3-[methyl-(7-nitrobenzo[1,2,5]oxadia-zol-4-yl)-amino]-propyl}-butane-1,4-diamine, in which:
R₁ = 4-NO₂, R₂ = methyl, a = 3, b = 4, c = 3, d = e = 1.

8. Compound according to claim 2, N-(3-aminopropyl)-N'-methyl-N'-(7-nitrobenzo[1,2,5]oxa-diazol-4-yl)-butane-1,4-diamine, in which: R₁ = 4-NO₂, R₂ = methyl, a = 0, b = 4, c = 3, d = 0, e = 1.

9. Compound according to claim 2, N-(3-aminopropyl)-N'-(7-nitrobenzo[1,2,5]oxadiazol-4-yl)-butane-1,4-diamine, in which: R₁ = 4-NO₂, R₂ = H, a = 0, b = 4, c = 3, d = 0, e = 1.

10. Compound according to claim 2, in which: R₁ = 4-NO₂, R₂ = methyl, a = 0, b = 3, c = 4, d = 0, e = 1.

11. Compound according to claim 2, in which: R₁ = 4-NO₂, R₂ = H, a = 0, b = 3, c = 4, d = 0, e = 1.

12. Compound according to claim 2, in which: R₁ = 4-NO₂, R₂ = methyl, a = 4, b = 0, c = 0, d = 1, e = 0.

13. Method to synthesize a compound of formula 1 as defined in claim 1, **characterized in that** it comprises the following steps:
a) Coupling a derivative of benzoxadiazole of formula 2 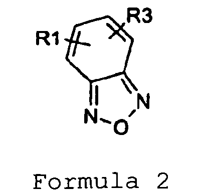 in which R₁ represents one or two nitro groups at position 4 and/or 6;
R₃ is a halogen substituent mobile at position 7, with a polyamine of formula 6 in which P is a protector group of the amine functions and in which a, b, c lie between 2 and 5, independently of each other, and in which d and e can independently be 0 or 1; 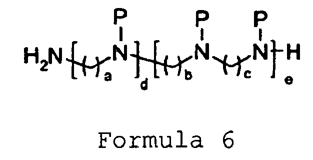
b) Alkylating the compound derived from the coupling reaction a) with an alkyl halide in the presence of a base in an inert solvent; and
c) Deprotecting the compound derived from step b) in an acid medium at room temperature to obtain the compound of formula 1.
